# EUROPÄISCHE PATENTSCHRIFT

(11) **EP 2 036 882 B1**
(45) Veröffentlichungstag und Bekanntmachung des Hinweises auf die Patenterteilung: **03.09.2014**
(21) Anmeldenummer: 08158822.0
(22) Anmeldetag: 24.06.2008
(51) Int. Cl.: C07C 233/36, C07C 235/10, C07C 271/02, A61K 31/205, A61K 31/164, A61P 17/00

(54) **Zwitterionische Verbindungen und deren Verwendung**
Zwitterionic connections and their use
Connexions zwitterioniques et leur utilisation

(30) Priorität: 23.08.2007 DE 102007040000
(43) Veröffentlichungstag der Anmeldung: 18.03.2009
(73) Patentinhaber: Evonik Degussa GmbH, 45128 Essen (DE)
(72) Erfinder: Herrwerth, Sascha, Dr., 45134 Essen (DE); Wenk, Hans, Henning, Dr., 45470 Mülheim a. d. Ruhr (DE); Grüning, Burghard, Dr., 45134 Essen (DE); Allef, Petra, Dr., 47807 Krefeld (DE); Begoihn, Uwe, 45359 Essen (DE)

(56) Entgegenhaltungen:
- EP-A- 0 656 346
- EP-A1- 0 418 697
- WO-A-97/01528
- DATABASE WPI Week 199437 Thomson Scientific, London, GB; AN 1994-299740 XP002511604 -& JP 06 228070 A (KAO CORP) 16. August 1994 (1994-08-16)
- DATABASE WPI Week 198802 Thomson Scientific, London, GB; AN 1988-011516 XP002511605 -& JP 62 275250 A (FUJI PHOTO FILM CO LTD) 30. November 1987 (1987-11-30)

## Beschreibung

### Gebiet der Erfindung:

Die vorliegende Erfindung betrifft zwitterionische Verbindungen und deren Herstellung, welche durch Carboxyalkylierung kurzkettiger Dialkylaminoalkylamide erhalten werden können.

### Stand der Technik:

Glycinatverbindungen, wie sie durch Carboxymethylierung der Dimethylaminopropylamide von Fettsäuren erhalten werden, sind seit langem bekannt. Sie sind unter der Bezeichnung "Amidopropylbetaine" als Tenside weit verbreitet (siehe z.B. Parfümerie und Kosmetik, 1996, 77(4), 244-248).

Bekannte Verbindungen dieser Stoffklasse sind bspw. von Cocosfettsäuren, hydrierten Cocosfettsäuren oder Cocosfettsäurefraktionen abgeleitet (z.B. Cocoamidopropylbetain). Sie finden breite Anwendung z.B. als Sekundärtenside in kosmetischen Präparaten (z.B. Duschgele, Shampoos, Flüssigseifen) sowie in Geschirrspülmitteln.

Charakteristisches Merkmal bekannter Amidopropylbetaine ist ihr tensidisches Verhalten, d. h. die Eigenschaft, die Oberflächenspannung wässriger Systeme zu verringern. Diese Eigenschaft wird durch die molekulare Struktur verursacht, nämlich durch den hydrophoben, langkettigen Fettsäurerest einerseits, und die hydrophile, zwitterionische Dimethylammoniumglycinat-Gruppe andererseits. Die gängigen Fettsäurereste umfassen insbesondere solche, die in natürlichen Fetten, insbesondere Cocosfett oder hydriertem Cocosfett vorkommen, also gesättigte und ungesättigte C₈-C₁₈ Fettsäuren. Beschrieben wurden auch Amidopropylbetaine von Capronsäure (C6).

Viele zwitterionische Substanzen wie z.B. Aminoxide, Prolin, Ectoin oder Trimethylglycin sind sogenannte Kosmotrope, d. h. sie erhöhen die Struktur bzw. Ordnung von wässrigen Systemen. Viele dieser Stoffe sind bekannterweise Osmolyte, d. h. sie kontrollieren den Wassergehalt von Zellen und wirken sich dadurch stabilisierend aus.

Eine nachteilige Wirkung vieler Tenside ist die Reizung von Haut und Augen. Zwar ist das Reizpotential der Amidopropyldimethylglycinate geringer als bspw. das der Alkylsulfate oder Alkylethersulfate, jedoch sind auch sie nicht vollständig frei von möglichen Reizwirkungen.

Aufgabe der Erfindung war es daher, neue Verbindungen zur Verfügung zu stellen, die zwar die zwitterionische, hydrophile Kopfgruppe, aber keine zellschädigende Wirkung wie Tenside gemäß dem Stand der Technik, somit keine tensidischen Strukturen aufweisen und vorzugsweise somit Haut und Augen nicht reizen. Bevorzugst sollen diese zwitterionischen Verbindungen mit Verfahren hergestellt werden können, die eine großtechnische Produktion ermöglichen.

Die JP6228070 offenbart Carbocybetaine der allgemeinen Formel (I) 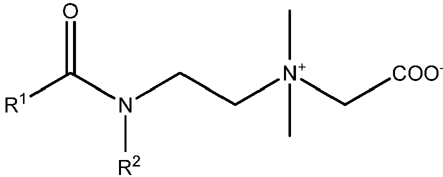 mit R¹= optional OH-substituierten 1-6C-Alkyl oder NR³R⁴-; R³, R⁴ = H oder optional OH-substituierten 1-6C-Alkyl und R² = H oder optional OH-substituierten 1-6C-Alkyl,
sowie ihre Verwendung in Haarkosmetika.

Die WO97/01528 offenbart ein Verfahren zur Herstellung von niedrigviskosen, wäßrigen Betainkonzentraten, bei dem man zunächst mehrwertige Carbonsäuren mit 2 bis 12 Kohlenstoffatomen mit Polyaminen amidiert und anschließend die resultierenden Amide in an sich bekannter Weise mit Halogencarbonsäuren oder deren Alkalisalzen betainisiert.

Die EP0656346 offenbart hochkonzentrierte Betaine, enthaltend 30 - 50 Gew.-% einer Verbindung der allgemeinen Formel (IV)

R³-CONH-(CH₂)ₘ-N⁺R⁴R⁵(CH₂)_{y}COO⁻

worin R³ der Alkylrest einer Fettsäure, welcher gegebenenfalls verzweigt, gegebenenfalls Mehrfachbindungen, gegebenenfalls Hydroxylgruppen enthalten kann,
R⁴ und R⁵ gleich oder verschiedene Alkylreste mit 1 - 4 C-Atomen bedeuten und m = 1 - 3 sein kann, 0,5 bis 5 Gew.-% mindestens einer Verbindung der allgemeinen Formel (I) 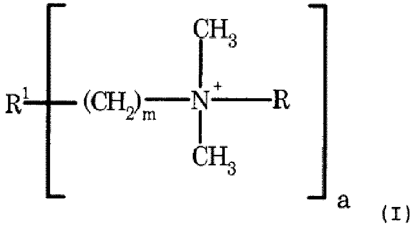
mit R = CH₃-, -CH₂COO K⁺, mit K⁺ = Ammonium oder Alkalimetallkation,
R¹- -N(R)₂ mit R = -CH2COO⁻ K⁺,
R¹ = OH mit R = CH₃ und m = 2,
R¹ = -NHCO-(CHR²)ₓ-CH₃ mit R² = H oder ein oder mehrere statistisch über die Kette verteilte C₁- bis C₃-Alkylgruppen und/oder OH-Gruppen und x = 0 bis 8,
R¹ = -NHCO-(CHR²)_{y}-CONH- mit R²= H oder ein oder mehrere statistisch über die Kette verteilte C₁- bis C₃-Alkylgruppen und mit a = 2 und y = 0 bis 8,
5 - 8 Gew.-% Natriumchlorid und ad 100 Gew.-% Wasser.

Die JP62275250 offenbart Amino-Verbindungen der Formel (HOCH₂CH₂)₃N und (C₂H₅)₂NCH₂CH₂OH.

Die EP0418697 offenbart pharmazeutische Zusammensetzungen für die intranasale Verabreichung von natürlichen oder synthetischen Calcitoninen, welche dadurch gekennzeichnet sind, dass sie eine langkettige N-subsitutierte Aminosäure enthalten.

### Beschreibung der Erfindung:

Überraschenderweise wurde gefunden, dass im Gegensatz zu den bekannten, oben beschriebenen Amidopropyldimethylglycinaten auf Basis langkettiger Fettsäuren die erfindungsgemäßen Verbindungen keine zellschädigende Wirkung aufweisen und nicht reizend sind, was vermutlich auf die fehlenden tensidischen Eigenschaften zurückzuführen ist.

Besonders überraschend wurde gefunden, dass erfindungsgemäße Verbindungen eine anti-inflammatorische Wirkung in Hautmodellen hervorrufen.

Gegenstand der vorliegenden Erfindung sind deshalb zwitterionische Verbindungen wie in Anspruch 1 bis 5 beschrieben, zur Verwendung als verschiedene Therapeutika wie in Ansprüchen 6 bis 8 beschrieben und ein Verfahren zu ihrer Herstellung wie in Anspruch 9 beschrieben.

Ein weiterer Vorteil der erfindungsgemäßen Verbindungen liegt in ihrem im Vergleich zu herkömmlich verwendeten Verbindungen kleinen Molekulargewicht, wodurch eine höhere Konzentration von ionischen bzw. zwitterionischen Strukturen erreicht werden kann.

Ein Vorteil des erfindungsgemäßen Verfahrens besteht in der Möglichkeit, die erfindungsgemäßen Verbindungen in hohen Konzentrationen ohne Gelbildung und somit ohne Viskositätsprobleme herzustellen.

Die erfindungsgemäßen Verbindungen und das Verfahren zur Herstellung dieser werden nachfolgend beispielhaft beschrieben, ohne dass die Erfindung auf diese beispielhaften Ausführungsformen beschränkt sein soll. Sind nachfolgend Bereiche, allgemeine Formeln oder Verbindungsklassen angegeben, so sollen diese nicht nur die entsprechenden Bereiche oder Gruppen von Verbindungen umfassen, die explizit erwähnt sind, sondern auch alle Teilbereiche und Teilgruppen von Verbindungen, die durch Herausnahme von einzelnen Werten (Bereichen) oder Verbindungen erhalten werden können. Werden im Rahmen der vorliegenden Beschreibung Dokumente zitiert, so soll deren Inhalt vollständig zum Offenbarungsgehalt der vorliegenden Erfindung gehören. Als "kurzkettige", zwitterionische, nicht tensidische Verbindungen sind im Folgenden solche zu verstehen, die gemäß Formel I ein X mit ≤ 5 Kohlenstoffatomen aufweisen. Als "länger- bzw. langkettige" zwitterionische, nicht tensidische Verbindungen sollen solche verstanden werden, die ein X mit > 5 Kohlenstoffatomen aufweisen. Alle angegebenen Prozent (%) sind wenn nicht anders angegeben Massenprozent.

Die vorliegende Erfindung beinhaltet Verbindungen der allgemeinen Formel I 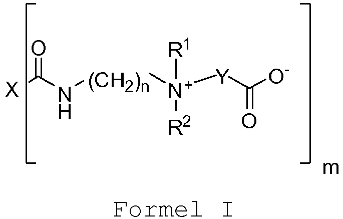 wobei
n = 1 bis 6, vorzugsweise 1 bis 3, bevorzugt 3 und m = 1 bis 4, vorzugsweise 1 oder 2 ist, und R¹ und R² unabhängig voneinander gleiche oder verschiedene, aliphatische Kohlenwasserstoffreste mit 1 bis 6 Kohlenstoffatomen, vorzugsweise C₁- bis C₃-Kohlenwasserstoffreste und bevorzugt CH₃-Reste sind, und Y ein zweibindiger Kohlenwasserstoffrest, vorzugsweise -CH₂- ist,
m = 1 und X = H ist. Eine besonders bevorzugte Verbindung ist die Ausführungsform der Formel I, bei der n=3, m=1, R¹ = R² = CH₃, Y = CH₂ und X = H ist.

Ein weiterer Gegenstand der Erfindung ist ein Arzneimittel bestehend aus einer wirksamen Menge mindestens einer der erfindungsgemäßen Verbindungen so wie die Verwendung der erfindungsgemäßen Verbindungen als Arzneimittel.

Wie in Beispielen 3.1 bis 3.4 gezeigt wirken erfindungsgemäße Verbindungen auf rekonstituierte humane Epidermis sowohl zell-protektiv als auch antiinflammatorisch. Somit sind erfindungsgemäße Verbindungen zur Verwendung als therapeutische Wirkstoffe Gegenstand der Erfindung.

Da der zellschützende und anti-apoptotische Effekt sich besonders gut auf Hautzellen darstellen lassen kann, ist eine bevorzugte Verwendung der erfindungsgemäßen Verbindungen eine zur Behandlung dermatologischer Erkrankungen, besonders bevorzugt zur Behandlung von inflammatorischer Hauterkrankungen.

Beispielhaft seien hier erwähnt: Psoriasis vulgaris, Pyoderma gangraenosum, Subkorneale Pustulose Sneddon Wilkinson, Atopische Dermatitis, Granuloma anulare, Pemphigus vulgaris, Vitiligo, Atopische Ekzem, Psoriasis, Neurodermitis, Urtikaria, Vaskulitis allergica, allergische Alveolitis, Kontaktekzem, seborrhöisches Ekzem, dishidrotisches Ekzem, Arzneiekzem, generelle allergische Hauterkrankungen, Alopecia areata, Alopecia totalis, Alopecia subtotalis, Alope cia universalis, Alopecia diffusa, Lupus erythematodes der Haut, Lichen planus, Dermatomyostis der Haut, Morphea, Sklerodermie, Psoriasis capitis, Psoriasis guttata, Psoriasis inversa, Alopecia areata Ophi- asis-Typ, androgenetische Alopezie, allergisches Kontaktekzem, irritatives Kontaktekzem, Kontaktekzem, Pemphigus foliaceus, Pemphigus vegetans, vernarbendes Schleimhautpemphigoid, bullöses Pemphgoid, Schleimhautpemphigoid, Dermatitis, Dermatitis herpetiformis duhring, Necrobiosis lipoidica, Erythema nodosum, Lichen vidal, Prurigo simplex, Prurigo nodularis, Prurigo acuta, lineare IgA Dermatose, polymorphe Lichtdermatose, Erythema solaris, Lichen sclerosus, eriorale Dermatitis, Exanthem der Haut, Arzneimittelexanthem, Purpura chronica progressiva, dihidrotisches Ekzem, Ekzem, fixes Arzneiexanthem, photoallergische Hautreaktion, Lichen simplex oder Graft versus Host-Disease.

Das erfindungsgemäße Arzneimittel kann durch jeden dem Fachmann bekannten Verabreichungsweg verabreicht werden, wie bspw. Intranasal, intravenös, intramuskulär, topisch und lokal, wobei bei Hauterkrankungen die topische und lokale Verabreichung bevorzugt ist. Die Therapie der Hauterkrankungen kann auf herkömmliche Weise, z. B. durch Verbände, Pflaster, Kompressen, rekonstruierbare Lyophilisate, als Pflaster oder Paste oder Gele erfolgen, die die erfindungsgemäßen Verbindungen enthalten, die erfindungsgemäßen Verbindungen können aber auch in Form von Liposomenkomplexen bzw. Goldpartikelkomplexe topisch und lokal im Bereich der erkrankten Hautfläche verabreicht werden. Weitere topische Darreichungen können beispielsweise in Form einer Lösung, einer Emulsion, einer Creme, einer Salbe, eines Schaums, eines Aerosol-Sprays, einer Gelmatrix, eines Schwamms als rekonstruierbares Lyophilisat, als Pflaster oder als Paste, Tropfen oder Waschungen erfolgen.

Die erfindungsgemäßen Verbindungen gemäß Formel I können z.B. mit dem nachfolgend beschriebenen, erfindungsgemäßen Verfahren hergestellt werden.

Dieses Verfahren zeichnet sich dadurch aus, dass in einem ersten Verfahrensschritt A eine Carbonsäure nach Formel II 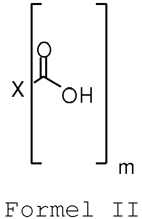 mit einem Amin der Formel III: 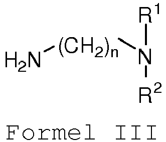 zu einem Amidamin gemäß Formel IV: 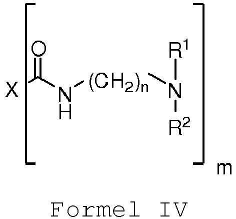 umgesetzt wird, wobei n = 1 bis 6 und m = 1 sind, und R¹ und R² unabhängig voneinander gleiche oder verschiedene, aliphatische Kohlenwasserstoffreste mit 1 bis 6 Kohlenstoffatomen sind, und m=1 und X = H ist und anschließend in Verfahrensschritt B das in A erhaltene Amidamin der Formel IV mit einer ω-Halogencarbonsäuren oder ihrem Salz, vorzugsweise Metallsalz, insbesondere Natriumsalz, die einen Säurerest gemäß Formel V aufweist 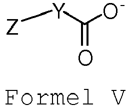 zur Verbindung der Formel I umgesetzt wird, wobei Z = ein Halogen und Y ein zweibindiger Kohlenwasserstoffrest ist.

Als Aminkomomponente können alle geeigneten Aminverbindungen, die die Bedingungen der Formel III erfüllen, eingesetzt werden. Vorzugsweise werden 3-(Diethylamino)propylamin, 2-(Diethylamino)ethylamin oder 2-(Dimethylamino)ethylamin eingesetzt. Besonders bevorzugt als Aminkomponente ist Dimethylaminopropylamin (DMAPA).

Bevorzugt wird im Schritt A des erfindungsgemäßen Verfahrens eine Säurekomponente gemäß Formel II mit einer Aminkomomponente gemäß Formel III bei einer Temperatur von 90 °C bis 220 °C, besonders bevorzugt bei einer Temperatur von ca. 180 °C zu einem Amidamin gemäß Formel IV umgesetzt. Besonders bevorzugt wird Verfahrensschritt A des erfindungsgemäßen Verfahrens unter Einsatz eines geeigneten Katalysators durchgeführt. Vorzugsweise werden als geeignete Katalysatoren starke Basenkatalysatoren wie z.B. Alkoholate eingesetzt, besonders bevorzugt werden Natriumethylat, Kaliumethylat, Natriummethylat und Kaliummethylat.

Das bei der Reaktion entstehende Wasser kann aus dem Produkt entfernt werden. Vorzugsweise wird das Wasser unter den Reaktionsbedingungen abdestilliert und so aus dem Produktgemisch entfernt. Insbesondere bei Temperaturen unterhalb ca. 130 °C ist das Anlegen eines Unterdruckes vorteilhaft, um die Wasserentfernung durch Abdestillieren zu beschleunigen.

Das nachfolgende Reaktionsschema zeigt einen möglichen Reaktionsverlauf des Verfahrensschrittes A.

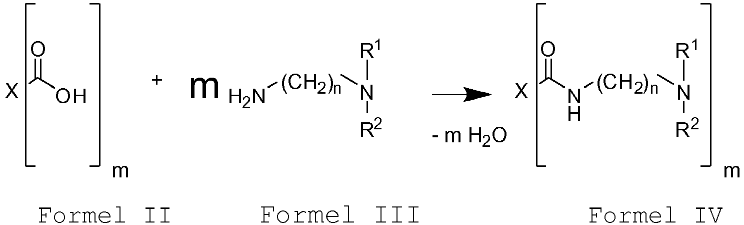

Da die in Verfahrensschritt A entstehenden Salzgemische zu Anfang der Reaktion fest sind, wird vorzugsweise in umgekehrter Reihenfolge verglichen zum Stand der Technik die Säurekomponente gemäß Formel II bei dem erfindungsgemäßen Verfahren zu der vorgelegten Aminkomponente gemäß Formel III zugegeben.

Bei der Herstellung kurzkettiger Amidamine gemäß Formel IV sollte der im Vergleich zu aus dem Stand der Technik bekannten Amidaminen längerkettiger Fettsäuren stark erhöhten Exothermie bei der Salzbildung zwischen Amin gemäß Formel III und Säure gemäß Formel II Rechnung getragen werden, die durch die niedrigen Molgewichte und dadurch höheren Stoffmengenkonzentrationen bedingt ist. Dazu können in einer bevorzugten Ausführungsform des erfindungsgemäßen Verfahrens in Verfahrensschritt A speziell angepasste Prozessparameter in der Form zur Anwendung kommen, dass die Zugabe der Carbonsäurekomponente zur Aminkomponente so langsam erfolgt, dass die Temperatur des Reaktionsgemisches während der Zugabe 130 °C, vorzugsweise 100 °C, nicht überschreitet. Bei höheren Temperaturen könnten durch das entstehende Wasser größere Mengen der Aminkomponente ausgetrieben werden, was sich negativ auf die Stöchiometrie der Einsatzkomponenten auswirken kann. Bevorzugt wird zur Einhaltung der genannten Temperaturbereiche gegengekühlt, um eine ökonomisch sinnvolle Dosiergeschwindigkeit zu erreichen.

Der Verfahrensschritt B kann in Gegenwart eines geeigneten Lösungsmittels in einer Menge, die die Rühr- und Pumpbarkeit des Reaktionsgemisches zu jedem Zeitpunkt des Verfahrens gewährleistet, erfolgen. Vorzugsweise erfolgt die Umsetzung in Gegenwart von Wasser als Lösungsmittel. Der Verfahrensschritt B wird vorzugsweise bei einer Temperatur von ca. 70-100 °C durchgeführt. Das als Nebenprodukt anfallende Halogenid Z kann aus der Reaktionslösung entfernt werden oder in dieser verbleiben. Soll das Halogenid entfernt werden, können z.B. Ausfällung mit einem geeigneten Lösungsmittel oder Dialyse zum Einsatz kommen. Bevorzugtes Lösungsmittel zur Fällung ist Ethanol.

In bevorzugten Ausführungsformen des erfindungsgemäßen Verfahrens verbleibt das Halogenid Z in der Lösung.

Als Monohalogencarbonsäure oder Monohalogencarbonsäuresalz mit einem Säurerest gemäß Formel V können alle Halogencarbonsäuren eingesetzt werden, deren Säurerest die für Formel V genannten Bedingungen erfüllen. Besonders bevorzugt als Monohalogencarbonsäuresalz gemäß Formel V ist das Monochloracetat.

Wie bereits in Verfahrensschritt A sollte auch im Verfahrensschritt B der durch die geringe Molmasse der kurzkettigen Amidaminkomponente bedingte, im Gegensatz zu den Verfahren des Standes der Technik stark erhöhte Exothermie Rechnung getragen werden. Daher erfolgt in Verfahrensschritt B des erfindungsgemäßen Verfahrens die Reaktion vorzugsweise in der Form, dass während und nach vollständiger Zugabe der Halogencarbonsäurekomponente zur Amidaminkomponente bis zum Abklingen der Wärmetönung die Reaktionstemperatur auf maximal ca. 70 °C gehalten wird, wobei ggf. gegengekühlt werden kann. Die nachfolgende Reaktion erfolgt vorzugsweise wenig unterhalb des Siedepunktes des Lösungsmittels, wobei bei Verwendung von Wasser als Lösungsmittels vorzugsweise Temperaturen im Bereich von 95-99 °C verwendet werden.

Das nachfolgende Reaktionsschema zeigt einen möglichen Reaktionsverlauf des Verfahrensschrittes B.

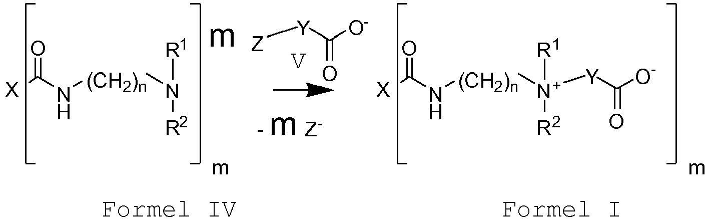

Die Umsetzung von Amidaminen gemäß Formel IV zu den entsprechenden Verbindungen gemäß Formel I erfolgt wie beschrieben vorzugsweise in einem Lösungsmittel. Die Amidamine werden bevorzugt in Konzentrationen von 3 bis 75 %, bevorzugt 5 bis 50% eingesetzt. Die in diesem Verfahrensschritt anfallende Lösung von Verbindungen gemäß Formel I kann mit oder ohne weitere Konzentrierungs- oder Entsalzungsschritte verwendet werden, z.B. zur Herstellung kosmetischer Präparate. In einer weiteren bevorzugten Ausführungsform des erfindungsgemäßen Verfahrens wird erfindungsgemäße Verbindung der Formel I durch Fällung der Halogenidkomponente mit einem geeigneten Lösungsmittel oder durch Dialyse entsalzt oder durch Destillation des gesamten oder eines Teiles des Lösungsmittels konzentriert. Bevorzugt werden die nach Verfahrensschritt B anfallenden Lösungen ohne weitere Aufarbeitungsschritte verwendet.

In bevorzugten Ausführungsformen erfindungsgemäßen Verfahrens wird das im Verfahrensschritt A im Überschuss eingesetzte Amin II vor der Umsetzung mit der Halogencarbonsäurekomponente in Verfahrensschritt B entfernt. Dies kann z.B. durch Abdestillieren bei vermindertem Druck erfolgen.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

### Beispiele:

### Beispiel 1.1: Herstellung der Verbindung 1.1

In einer 500 ml Rührapparatur mit Rückflusskühler und Stickstoffeinleitung wurden 100 g Ameisensäure vorgelegt und mit Stickstoff ca. 10 Minuten inertisiert. Dann wurden 225 ml 3-Dimethylaminopropylamin unter Rühren und fortlaufender Inertisierung mit Stickstoff zugegeben. Die Salzbildung war exotherm und das Gemisch heizte sich auf ca. 175 °C auf und wurde etwa 4 - 5 h bei dieser Temperatur gehalten. Während dieser Zeit wurde bei der Reaktion entstehendes Wasser über eine Kolonne aus dem Gemisch entfernt. Wenn anhand der Säurezahl ein Umsetzungsgrad von ca. 98% erreicht war, wurde überschüssiges DMAPA mittels Vakuumdestillation entfernt. Der Gehalt an tertiärem Stickstoff betrug im gereinigten Endprodukt 10,4%.

### Vergleichsbeispiel 1.2: Herstellung der Verbindung 1.2

In einer 500 ml Rührapparatur mit Rückflusskühler und Stickstoffeinleitung wurden 133 g Milchsäure vorgelegt und mit Stickstoff ca. 10 Minuten inertisiert. Dann wurden 188 ml 3-Dimethylaminopropylamin unter Rühren und fortlaufender Inertisierung mit Stickstoff zugegeben. Die Salzbildung war exotherm und das Gemisch heizt sich auf ca. 150 °C auf und wurde etwa 4 - 5 h bei einer Temperatur von 175 °C gehalten. Während dieser Zeit wurde bei der Reaktion entstehendes Wasser über eine Kolonne aus dem Gemisch entfernt. Wenn anhand der Säurezahl ein Umsetzungsgrad von ca. 98% erreicht war, wurde überschüssiges DMAPA mittels Vakuumdestillation entfernt. Der Gehalt an tertiärem Stickstoff betrug im gereinigten Endprodukt 8,14%.

### Vergleichsbeispiel 1.3: Herstellung der Verbindung 1.3

In einer 500 ml Rührapparatur mit Rückflusskühler und Stickstoffeinleitung wurden 148 g Propionsäure vorgelegt und mit Stickstoff ca. 10 Minuten inertisiert. Dann wurden 280 ml 3-Dimethylaminopropylamin unter Rühren und fortlaufender Inertisierung mit Stickstoff zugegeben. Die Salzbildung war exotherm und das Gemisch heizt sich auf ca. 150 °C auf und wurde etwa 4 - 5 h bei einer Temperatur von 175 °C gehalten. Während dieser Zeit wurde bei der Reaktion entstehendes Wasser über eine Kolonne aus dem Gemisch entfernt. Wenn anhand der Säurezahl ein Umsetzungsgrad von ca. 98% erreicht war, wurde überschüssiges DMAPA mittels Vakuumdestillation entfernt. Der Gehalt an tertiärem Stickstoff betrug im gereinigten Endprodukt 8,91%.

### Vergleichsbeispiel 1.4: Herstellung der Verbindung 1.4

In einer 500 ml Rührapparatur mit Rückflusskühler und Stickstoffeinleitung wurden 90 g Oxalsäure vorgelegt. Dann wurden 328 ml 3-Dimethylaminopropylamin unter Rühren und fortlaufender Inertisierung mit Stickstoff zugegeben. Nach dem Schmelzen des entstandenen Feststoffes war die Salzbildung exotherm und das Gemisch heizt sich auf ca. 150 °C auf und wurde etwa 4 - 5 h bei einer Temperatur von 175 °C unter Stickstoff gehalten. Während dieser Zeit wurde bei der Reaktion entstehendes Wasser über eine Kolonne aus dem Gemisch entfernt. Wenn anhand der Säurezahl ein Umsetzungsgrad von ca. 98% erreicht war, wurde überschüssiges DMAPA mittels Vakuumdestillation entfernt. Der Gehalt an tertiärem Stickstoff betrug im gereinigten Endprodukt 12,5%.

### Vergleichsbeispiel 1.5: Herstellung der Verbindung 1.5

In einer 500 ml Rührapparatur mit Rückflusskühler und Stickstoffeinleitung wurden 104 g Malonsäure vorgelegt. Dann wurden 280 ml 3-Dimethylaminopropylamin unter Rühren und fortlaufender Inertisierung mit Stickstoff zugegeben. Nach dem Schmelzen des entstandenen Feststoffes war die Salzbildung exotherm und das Gemisch heizt sich auf ca. 140 °C auf und wurde etwa 4 - 5 h bei einer Temperatur von 175 °C unter Stickstoff gehalten. Während dieser Zeit wurde bei der Reaktion entstehendes Wasser über eine Kolonne aus dem Gemisch entfernt. Wenn anhand der Säurezahl ein Umsetzungsgrad von ca. 98% erreicht war, wurde überschüssiges DMAPA mittels Vakuumdestillation entfernt. Der Gehalt an tertiärem Stickstoff betrug im gereinigten Endprodukt 9,84%.

### Vergleichsbeispiel 1.6: Herstellung der Verbindung 1.6

In einer 500 ml Rührapparatur mit Rückflusskühler und Stickstoffeinleitung wurden 76 g Glycolsäure vorgelegt. Dann wurden 135 ml 3-Dimethylaminopropylamin unter Rühren und fortlaufender Inertisierung mit Stickstoff zugegeben. Nach dem Schmelzen des entstandenen Feststoffes war die Salzbildung exotherm und das Gemisch heizt sich auf ca. 140 °C auf und wurde etwa 4 - 5 h bei einer Temperatur von 175 °C unter Stickstoff gehalten. Während dieser Zeit wurde bei der Reaktion entstehendes Wasser über eine Kolonne aus dem Gemisch entfernt. Wenn anhand der Säurezahl ein Umsetzungsgrad von ca. 98% erreicht war, wurde überschüssiges DMAPA mittels Vakuumdestillation entfernt. Der Gehalt an tertiärem Stickstoff betrug im gereinigten Endprodukt 8,96%.

### Beispiel 2.1: Herstellung der Verbindung 2.1

In einem 500 ml Vierhalskolben, ausgerüstet mit Rührer, Thermometer, Rückflusskühler und Tropftrichter wurden 91 g Na-Monochloracetat und 191 g Wasser eingewogen und auf 40 °C erwärmt. Es wurden 100 g des Amidamins aus Beispiel 1.1 zugegeben und das Reaktionsgemisch bis zum Abklingen der Wärmetönung bei einer Temperatur von 70 °C gehalten. Dann erhitzte man auf 98 °C. Nach ca. 7 h lag der Gehalt an restlichem Amidamin unter 0,5%.
Es wurden 382 g einer wässrigen Lösung der folgenden Zusammensetzung erhalten:

| | |
|---|---|
| Verbindung 2.1 | 38,1% |
| NaCl: | 11,9% |
| Wasser: | 50 % |
| Aussehen: | flüssig, klar |

### Vergleichsbeispiel 2.2: Herstellung der Verbindung 2.2

In einem 500 ml Vierhalskolben, ausgerüstet mit Rührer, Thermometer, Rückflusskühler und Tropftrichter wurden 70 g Na-Monochloracetat und 170 g Wasser eingewogen und auf 40 °C erwärmt. Es wurden 100 g des Amidamins aus Beispiel 1.2 zugegeben. Dann erwärmte man das Reaktionsgemisch auf 70 °C und hält die Temperatur bis zum Abklingen der Wärmetönung. Anschließend erhitzte man auf 98 °C. Nach ca. 7h lag der Gehalt an restlichem Amidamin unter 0,5%.
Es wurden 340 g einer wässrigen Lösung der folgenden Zusammensetzung erhalten:

| | |
|---|---|
| Verbindung 2.2: | 39,7% |
| NaCl: | 10,3% |
| Wasser: | 50 % |
| Aussehen: | flüssig, klar |

### Vergleichsbeispiel 2.3: Herstellung der Verbindung 2.3

In einem 1000 ml Vierhalskolben, ausgerüstet mit Rührer, Thermometer, Rückflusskühler und Tropftrichter wurden 153 g Na-Monochloracetat und 353 g Wasser eingewogen und auf 40 °C erwärmt. Es wurden 200 g des Amidamins aus Beispiel 1.3 zugegeben. Dann erwärmte man das Reaktionsgemisch auf 70 °C und hält die Temperatur bis zum Abklingen der Wärmetönung. Anschließend erhitzte man auf 98 °C. Nach ca. 7h lag der Gehalt an restlichem Amidamin unter 0,5%.
Es wurden 706 g einer wässrigen Lösung der folgenden Zusammensetzung erhalten:

| | |
|---|---|
| Verbindung 2.3: | 39,2% |
| NaCl: | 10,8% |
| Wasser: | 50 % |
| Aussehen: | flüssig, klar |

### Vergleichsbeispiel 2.4: Herstellung der Verbindung 2.4

In einem 500 ml Vierhalskolben, ausgerüstet mit Rührer, Thermometer, Rückflusskühler und Tropftrichter wurden 107 g Na-Monochloracetat und 207 g Wasser eingewogen und auf 40 °C erwärmt. Es wurden 100 g des Amidamins aus Beispiel 1.4 zugegeben. Dann erwärmte man das Reaktionsgemisch auf 70 °C und hält die Temperatur bis zum abklingen der Wärmetönung. Anschließend erhitzte man auf 98 °C. Nach ca. 7h lag der Gehalt an restlichem Amidamin unter 0,5%.
Es wurden 414 g einer wässrigen Lösung der folgenden Zusammensetzung erhalten:

| | |
|---|---|
| Verbindung 2.4: | 35,9% |
| NaCl: | 14,1% |
| Wasser: | 50 % |
| Aussehen: | flüssig, klar |

### Vergleichsbeispiel 2.5: Herstellung der Verbindung 2.5

In einem 500 ml Vierhalskolben, ausgerüstet mit Rührer, Thermometer, Rückflusskühler und Tropftrichter wurden 85 g Na-Monochloracetat und 185 g Wasser eingewogen und auf 40 °C erwärmt. Es wurden 100 g des Amidamins aus Beispiel 1.5 zugegeben. Dann erwärmte man das Reaktionsgemisch auf 70 °C und hält die Temperatur bis zum Abklingen der Wärmetönung. Anschließend erhitzte man auf 98 °C. Nach ca. 7h lag der Gehalt an restlichem Amidamin unter 0,5%.
Es wurden 370 g einer wässrigen Lösung der folgenden Zusammensetzung erhalten:

| | |
|---|---|
| Verbindung 2.5: | 38,5% |
| NaCl: | 11,5% |
| Wasser: | 50 % |
| Aussehen: | flüssig, klar |

### Vergleichsbeispiel 2.6: Herstellung der Verbindung 2.6

In einem 1000 ml Vierhalskolben, ausgerüstet mit Rührer, Thermometer, Rückflusskühler und Tropftrichter wurden 115 g Na-Monochloracetat und 265 g Wasser eingewogen und auf 40 °C erwärmt. Es wurden 150 g des Amidamins aus Beispiel 1.6 zugegeben. Dann erwärmte man das Reaktionsgemisch auf 70 °C und hält die Temperatur bis zum Abklingen der Wärmetönung. Anschließend erhitzte man auf 98 °C. Nach ca. 7h lag der Gehalt an restlichem Amidamin unter 0,5%.
Es wurden 530 g einer wässrigen Lösung der folgenden Zusammensetzung erhalten:

| | |
|---|---|
| Verbindung 2.6: | 38,5% |
| NaCl: | 11,5% |
| Wasser: | 50 % |
| Aussehen: | flüssig, klar |

### Effektivitätsbeispiele für erfindungsgemäße Verbindungen

Um die therapeutischen und hautpflegenden Eigenschaften der Verbindung 2.1 und der nicht erfindungsgemäßen Verbindungen 2.2 bis 2.6 charakterisieren zu können, wurden verschiedene in-vitro-Tests an Hautmodellen (rekonstituierte humane Epidermis, Firma: SkinEthic) durchgeführt.

### Beispiel 3.1 Laktatdehydrogenase-Freisetzung (LDH-Freisetzung)

Das Auftreten von LDH im Zellkulturmedium ist ein sicheres Anzeichen für die Schädigung der zytoplasmatischen Membran der Zellen und damit einer Schädigung der epidermalen Zellschicht. Weiterhin ist bekannt, dass ein Austritt dieses Enzyms für die Zelle den *"point of no return"* darstellt, also eine Irreversibilität der Schädigung anzeigt.

Die Bestimmung der LDH-Konzentration erfolgte mit einem kommerziell erhältlichen Testkit (LDH-Testkit, Roche Diagnostics, Mannheim, Deutschland).

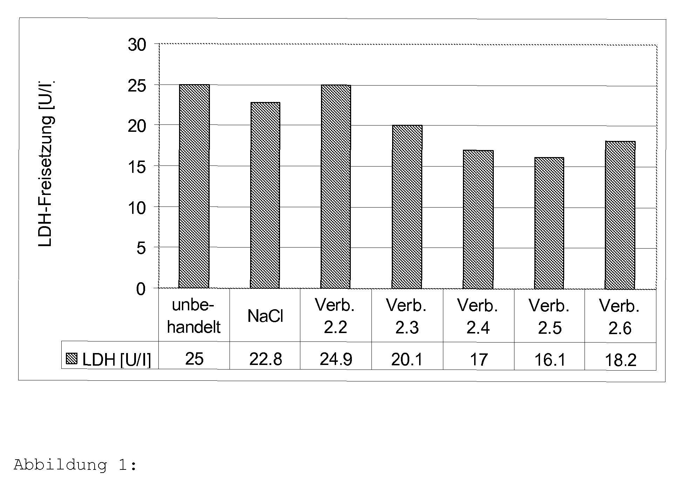

Die Testformulierung wurde zweimal im Abstand von 24h auf die Hautmodelle appliziert. Abbildung 1 gibt die LDH-Freisetzung 24h nach der letzten Applikation wieder.

Testformulierung 3.1: Es wurde eine 4%ige wässrige Lösung der erfindungsgemäßen Verbindungen appliziert. Da die Zwitterionische, nicht tensidische Verbindungen je 1% Aktivsubstanz ca. 0,3% Kochsalz enthalten, wurde zusätzlich die entsprechende Kochsalzkonzentration getestet.

Durch die Applikation der zwitterionische Verbindungen wurde die LDH-Freisetzung aus den Zellen nicht verändert oder sogar im Vergleich zum unbehandelten Hautmodell geringfügig kleiner. Das bedeutet, dass die erfindungsgemäßen Verbindungen die Zellmembran nicht angreifen und nicht zellschädigend wirken sondern ganz im Gegenteil sogar die Zellen und ihre Proliferation unterstützen.

### Beispiel 3.2 LDH-Freisetzung nach Schädigung der Zellen mit SDS:

Sodiumdodecylsulfat (SDS) ist bekannt dafür, dass es die Zellmembran angreift und zu einer erhöhten LDH-Freisetzung führt. Mit dem im Folgenden beschriebenen Versuch sollte untersucht werden, inwieweit Verbindung 2.1 die Zellen nach einer Schädigung mit SDS schützen kann.
Die Hautmodelle wurden 40 min mit SDS geschädigt. Anschließend wurde die Testformulierung, eine O/W-Creme mit 1 oder 4 % Verbindung 2.1, aufgetragen. Die LDH-Freisetzung wurde 24 und 48h nach Applikation der Testformulierung gemessen.

### Testformulierung 3.2:

| | |
|---|---|
| Polyglyceryl-3 Methylglucose Distearat | 3,0 % |
| Glyceryl Stearate | 2,0 % |
| Stearyl Alcohol | 1,0 % |
| Cetearyl Ethylhexanoate | 5,0 % |
| Mineral Oil | 14,0 % |
| Verbindung 2.1 | 1,0/4,0% |
| Wasser | ad 100,0% |

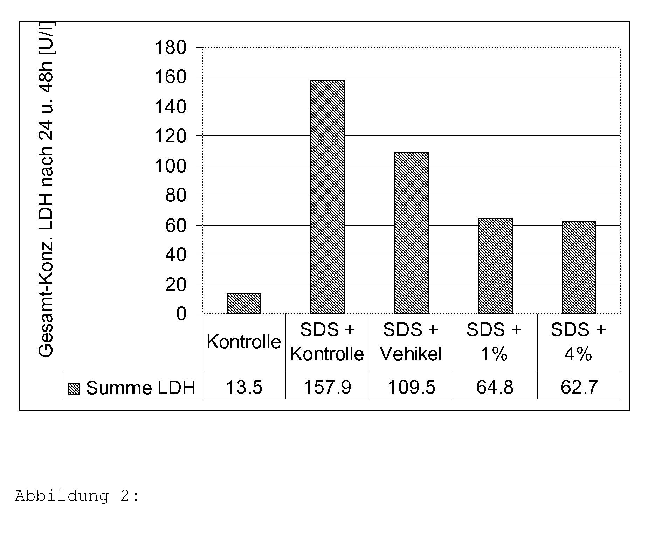

Abbildung 2 gibt die Gesamtkonzentration LDH nach 24 und 48h wieder.

Durch die Schädigung mit SDS stieg die LDH-Freisetzung wie erwartet stark an. Dieser Anstieg wurde deutlich reduziert, wenn direkt nach der Schädigung die Testformulierung appliziert wurde. Ein positiver Effekt war bereits beim Vehikel erkennbar, er verstärkte sich aber nochmal deutlich, wenn die Formulierung Verbindung 2.1 enthielt.

Dabei scheint bereits 1% der erfindungsgemäßen Verbindung ausreichend zu sein, da mit 4% keine deutliche Steigerung der Wirksamkeit erkennbar war.

### Beispiel 3.3 Il-1α-Freisetzung nach Schädigung mit SDS:

IL1-α ist ein Botenstoff, der bei entzündlichen Reaktionen im Körper eine zentrale Rolle spielt. Natrium Laurylsulfat (SDS) ist ein hautreizendes Tensid, welches eine irritative Kontaktdermatitis beim Menschen hervorrufen kann, als Modellreizstoff bei Probandenstudien verwendet wird und unter anderem die Freisetzung von IL-1α induziert. Die Bestimmung der IL-1 α-Konzentration erfolgte mit einem kommerziell erhältlichen Test-Kit (Human IL-1α Immunoassay, R&D Systems GmbH, Wiesbaden, Deutschland).

Die Testformulierung, eine 4%ige wässrige Lösung der kurzkettigen, zwitterionischen Verbindungen, wurde auf die Hautmodelle appliziert. 24h nach der Applikation wurde 40 min mit 0,25%iger SDS-Lösung geschädigt. Anschließend wurde die Testformulierung ein zweites Mal appliziert. Nach weiteren 24h Inkubationszeit erfolgte die Bestimmung des freigesetzten Cytokins IL-1α.

Da die Testlösungen je 1% Aktivsubstanz ca. 0,3% NaCl enthalten, wurde auch eine entsprechend konzentrierte Kochsalzlösung untersucht.

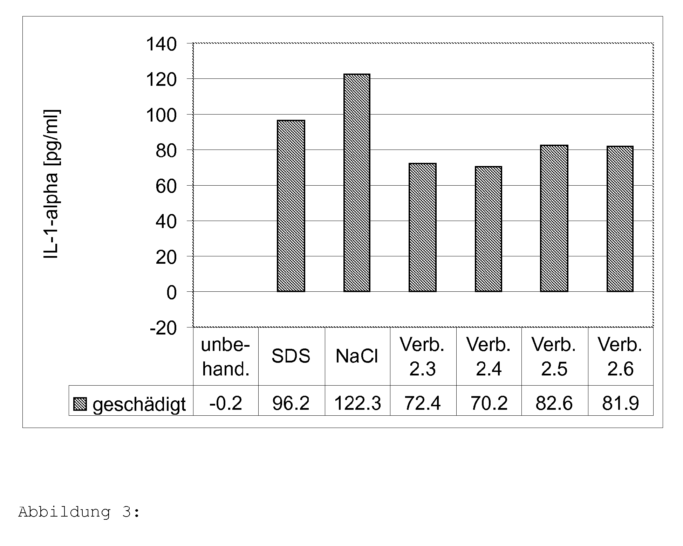

Abbildung 3 zeigt die Messwerte der IL-1α-Konzentration 24h nach Schädigung.

Alle getesteten zwitterionischen, nicht tensidischen Verbindungen reduzierten die Freisetzung des Entzündungsmarkers IL-1α, d.h. man kann davon ausgehen, dass die kurzkettigen, zwitterionischen Verbindungen entzündungshemmende Eigenschaften besitzen.

### Beispiel 3.4 Entzündungshemmende Wirkung einer O/W-Creme mit Verbindung 2.1:

Es sollte untersucht werden, ob sich der entzündungshemmende Effekt der erfindungsgemäßen Verbindungen auch bei Anwendung aus einer kosmetischen Formulierung zeigt. Dazu wurden Hautmodelle mit SDS geschädigt. Anschließend wurde die Testformulierung appliziert. 24 und 48h nach der Applikation wurde die IL-1α-Konzentration ermittelt.

### Testformulierung:

| | |
|---|---|
| Polyglyceryl-3 Methylglucose Distearat | 3,0 % |
| Glyceryl Stearate | 2,0 % |
| Stearyl Alcohol | 1,0 % |
| Cetearyl Ethylhexanoate | 5,0 % |
| Mineral Oil | 14,0 % |
| Verbindung 2.1 | 1,0/4,0% |
| Wasser | ad 100,0% |

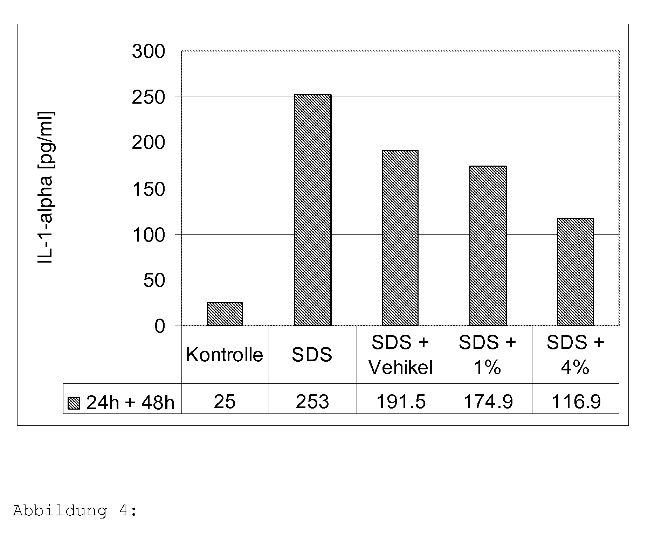

Abbildung 4 gibt die summierte IL-1α-Konzentration nach 24 und 48h wieder.

Wie erwartet stieg durch die Schädigung mit SDS die Bildung des Cytokins IL-1α stark an. Dieser Anstieg wurde konzentrationsabhänigig durch Zugabe von Verbindung 2.1 stärker reduziert, so dass sich auch bei Anwendung der erfindungsgemäßen Verbindungen aus einer O/W-Emulsion eine deutlich entzündungshemmende Wirkung zeigt.

## Patentansprüche

1. Verbindungen der allgemeinen Formel I 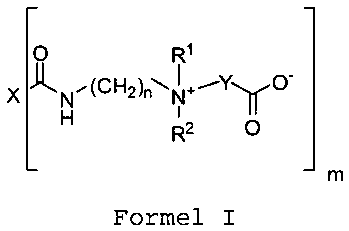 wobei
n = 1 bis 6 und
m = 1 und
R¹ und R² unabhängig voneinander gleiche oder verschiedene, aliphatische Kohlenwasserstoffreste mit 1 bis 6 Kohlenstoffatomen sind und
Y ein zweibindiger Kohlenwasserstoffrest und
X H
ist.

2. Verbindungen gemäß Anspruch 1, **dadurch gekennzeichnet, dass** n = 3 ist.

3. Verbindungen gemäß Anspruch 1 oder 2, **dadurch gekennzeichnet, dass** R¹ = R² = CH₃ ist.

4. Verbindungen gemäß mindestens einem der Ansprüche 1 bis 3, **dadurch gekennzeichnet, dass** Y = CH₂ ist.

5. Verbindung gemäß Anspruch 1, **dadurch gekennzeichnet, dass** n = 3, m = 1, R¹ = R² = CH₃, Y = CH₂ und X = H ist.

6. Verbindungen gemäß mindestens einem der Ansprüche 1 bis 5 zur Verwendung als therapeutische Wirkstoffe.

7. Verbindungen gemäß mindestens einem der Ansprüche 1 bis 5 zur Behandlung dermatologischer Erkrankungen.

8. Verbindungen gemäß mindestens einem der Ansprüche 1 bis 5 zur Behandlung inflammatorischer Hauterkrankungen.

9. Verfahren zur Herstellung von Verbindungen gemäß mindestens einem der Ansprüche 1 bis 5, **gekennzeichnet durch** die Schritte
A. Umsetzen einer Carbonsäure nach Formel II 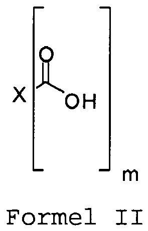 mit einem Amin der Formel III 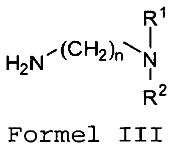 zu einem Amidamin gemäß Formel IV 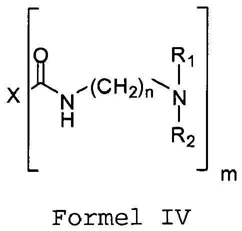 wobei
n = 1 bis 6 und
m = 1 und
R¹ und R² unabhängig voneinander gleiche oder verschiedene, aliphatische Kohlenwasserstoffreste mit 1 bis 6 Kohlenstoffatomen sind und
X H ist, und
B. Umsetzen des in A erhaltenen Amidamins der Formel IV mit einer ω-Halogencarbonsäure oder ihrem Salz, die einen Säurerest gemäß Formel V aufweist 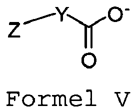 zur Verbindung der Formel I
wobei
Z = ein Halogen und
Y ein zweibindiger Kohlenwasserstoffrest ist.

## Claims

1. Compounds of the general formula I 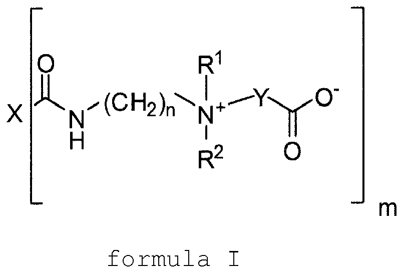 where
n = 1 to 6 and
m = 1 and
R¹ and R², independently of one another, are identical or different, aliphatic hydrocarbon radicals having 1 to 6 carbon atoms and
Y is a divalent hydrocarbon radical and
X is H.

2. Compounds according to Claim 1, **characterized in that** n = 3.

3. Compounds according to Claim 1 or 2, **characterized in that** R¹ = R² - CH₃.

4. Compounds according to at least one of Claims 1 to 3, **characterized in that** Y = CH₂.

5. Compound according to Claim 1, **characterized in that** n = 3, m = 1, R¹ = R² = CH₃, Y = CH₂ and X = H.

6. Compounds according to at least one of Claims 1 to 5 for use as therapeutic active ingredients.

7. Compounds according to at least one of Claims 1 to 5 for the treatment of dermatological disorders.

8. Compounds according to at least one of Claims 1 to 5 for the treatment of inflammatory skin disorders.

9. Process for the preparation of compounds according to at least one of Claims 1 to 5, **characterized by** the steps
A. reaction of a carboxylic acid according to formula II 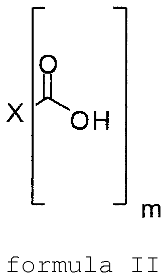 with an amine of the formula III 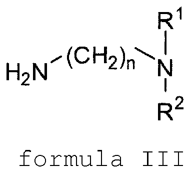 to give an amidoamine according to formula IV 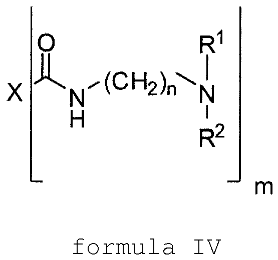 where
n = 1 to 6 and
m = 1 and
R¹ and R², independently of one another, are identical or different, aliphatic hydrocarbon radicals having 1 to 6 carbon atoms and
X is H, and
B. reaction of the amidoamine of the formula IV obtained in A with an ω-halocarboxylic acid or its salt which has an acid radical according to formula V 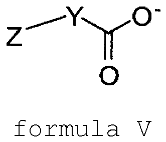 to give the compound of the formula I where
Z = a halogen and
Y is a divalent hydrocarbon radical.

## Revendications

1. Composés de formule générale I 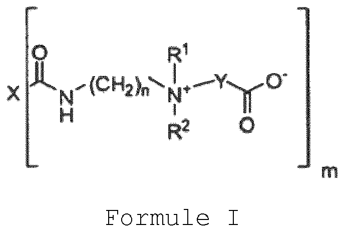 dans laquelle
n = 1 à 6 et
m = 1 et
R¹ et R² représentent indépendamment l'un de l'autre des radicaux hydrocarbonés aliphatiques identiques ou différents contenant 1 à 6 atomes de carbone, et
Y représente un radical hydrocarboné bivalent et
X représente H.

2. Composés selon la revendication 1, **caractérisés en ce que** n = 3.

3. Composés selon la revendication 1 ou 2, **caractérisés en ce que** R¹ = R² = CH₃.

4. Composés selon au moins l'une quelconque des revendications 1 à 3, **caractérisés en ce que** Y = CH₂.

5. Composé selon la revendication 1, **caractérisé en ce que** n = 3, m = 1, R¹ = R² = CH₃, Y = CH₂ et X = H.

6. Composés selon au moins l'une quelconque des revendications 1 à 5, destinés à une utilisation en tant qu'agents actifs thérapeutiques.

7. Composés selon au moins l'une quelconque des revendications 1 à 5, pour le traitement de maladies dermatologiques.

8. Composés selon au moins l'une quelconque des revendications 1 à 5, pour le traitement de maladies cutanées inflammatoires.

9. Procédé de fabrication de composés selon au moins l'une quelconque des revendications 1 à 5, **caractérisé par** les étapes suivantes :
A. la mise en réaction d'un acide carboxylique de formule II 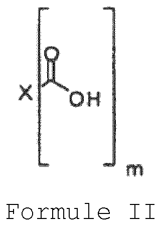 avec une amine de formule III 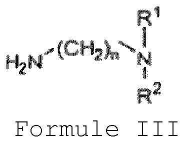 pour former une amide-amine de formule IV 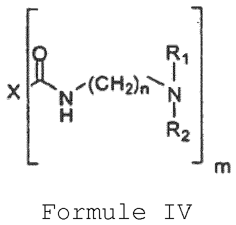 dans laquelle
n = 1 à 6 et
m = 1 et
R¹ et R² représentent indépendamment l'un de l'autre des radicaux hydrocarbonés aliphatiques identiques ou différents contenant 1 à 6 atomes de carbone, et
X représente H, et
B. la mise en réaction de l'amide-amine de formule IV obtenue en A avec un acide ω-halogénocarboxylique ou son sel, qui comprend un radical acide de formule V 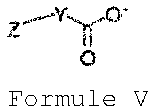 pour former le composé de formule I
dans laquelle
Z = un halogène et
Y = un radical hydrocarboné bivalent.
